(19) Europäisches Patentamt
European Patent Office
Office européen des brevets

(11) Numéro de publication: **0 298 818 B1**

(12) # FASCICULE DE BREVET EUROPEEN

(45) Date de publication de fascicule du brevet: **26.08.92**

(51) Int. Cl.$^5$: **C07D 207/48**, A61K 31/40

(21) Numéro de dépôt: **88401588.4**

(22) Date de dépôt: **23.06.88**

Le dossier contient des informations techniques présentées postérieurement au dépôt de la demande et ne figurant pas dans le présent fascicule.

(54) **Dérivés de la 1-benzène-sulfonyl 2-oxo 5-alkylthio pyrrolidine, leur procédé et intermédiaires de préparation, leur application comme médicaments et les compositions les renfermant.**

(30) Priorité: **29.06.87 IT 2109887**

(43) Date de publication de la demande:
**11.01.89 Bulletin 89/02**

(45) Mention de la délivrance du brevet:
**26.08.92 Bulletin 92/35**

(84) Etats contractants désignés:
**CH DE ES FR GB GR IT LI NL**

(56) Documents cités:
**EP-A- 0 138 721**

(73) Titulaire: **ROUSSEL-UCLAF**
**35, Boulevard des Invalides**
**F-75007 Paris(FR)**

(72) Inventeur: **Toja, Emilio**
**Via Plezzo 80**
**Milano(IT)**
Inventeur: **Zirotti, Carlo**
**Via 2, Giugno 12**
**Arona (NO)(IT)**
Inventeur: **Barzaghi, Fernando**
**Via Monteverdi 21**
**I-20052 Monza - Milan(IT)**
Inventeur: **Galliani, Giulio**
**13, Via Silva**
**I-1 Monza (MI)(IT)**

(74) Mandataire: **Tonnellier, Marie-José et al**
**111, route de Noisy B.P. no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

L'invention concerne de nouveaux dérivés de 1-benzènesulfonyl 2-oxo 5-alkylthio pyrrolidine, leurs procédé et intermédiaires de préparation, leur application comme médicaments et les compositions les renfermant.

Le brevet européen EP-A-138 721 décrit des 4-hydroxy, 4-alkyloxy ou 4-acyloxy 1-benzènesulfonyl 2-oxopyrrolidines pouvant être utilisés dans le traitement des troubles de la mémoire ; on vient de découvrir des composés de structure chimique apparentée présentant d'intéressantes propriétés biologiques.

L'invention a pour objet les composés répondant à la formule générale (I) :

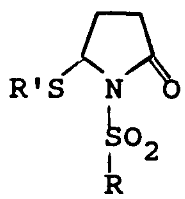

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyrannyle, pyridyle, benzofurannyle, isobenzofurannyle, chromanyle, isochromanyle, chroményle, xanthényle, phénoxathiényle, oxazolyle, isoxazolyle, furazanyle, phénoxazinyle, thiéno[2,3-b]furannyle, 2H-furo[3,2-b]pyrannyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, comme le fluor, le chlore, le brome, les groupements $CF_3$, $SCF_3$, $OCF_3$, $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone.

Par radical alcoyle, on entend de préférence un radical renfermant de 1 à 6 atomes de carbone, par exemple, le radical méthyle, éthyle,n-propyle, isopropyle, n-butyle, isobutyle, terbutyle, n-pentyle, n-hexyle, cyclopropyle, cyclobutyle, cyclopentyle ou cyclohexyle.

Par radical alcényle, on entend de préférence un radical éthényle, propényle, butényle.

Par radical acyle, on entend de préférence un radical acétyle, propionyle ou butyryle.

Lorsque le radical R est substitué par un ou plusieurs radicaux alcoyles linéaires, ramifiés ou cycliques, saturés ou insaturés, il s'agit de préférence du (ou de plusieurs) radical méthyle, éthyle, propyle ou isopropyle, éthényle ou éthynyle.

Lorsque le radical R est substitué par un ou plusieurs atomes d'halogène, il s'agit de préférence d'un ou plusieurs atomes de fluor, de chlore ou de brome.

L'invention a plus particulièrement pour objet les composés de formule (I) dans lesquels R représente un radical phényle éventuellement substitué ainsi que ceux dans lesquels R' représente un radical alcoyle linéaire, ramifié ou cyclique, renfermant jusqu'à 8 atomes de carbone comme par exemple un radical éthyle ou isopropyle.

L'invention a tout spécialement pour objet les composés dont la préparation est donnée plus loin dans la partie expérimentale.

Parmi ces composes, on peut citer comme composés préférés, les composés des exemples 1 et 3.

Les composés de l'invention présentent d'intéressantes propriétés pharmacologiques : ils retardent l'extinction de la réponse d'évitement conditionné, ils retardent la disparition de la réponse apprise. Ils favorisent l'attention, la vigilance et la mémorisation.

L'invention a donc pour objet les produits de formule (I) en tant que médicaments, utiles notamment dans le traitement des asthénies intellectuelles ou nerveuses, des défaillances de la mémoire, de la sénescence, du surmenage intellectuel.

L'invention a plus particulièrement pour objet en tant que médicament, les produits préférés mentionnés précédemment et notamment les produits des exemples 1 et 3.

La posologie usuelle est variable selon l'affection en cause, le sujet traité et la voie d'administration, elle peut être comprise entre 50 mg et 3000 mg/jour, par exemple entre 150 et 1500 mg/jour en une ou

plusieurs prises pour le produit de l'exemple 1 administré par voie orale.

La présente invention a également pour objet les compositions pharmaceutiques renfermant comme principe actif au moins un produit de formule (I).

Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) :

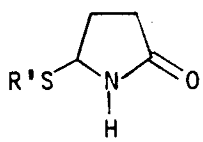

(II)

dans laquelle R' conserve la même signification que précédemment, à l'action d'un composé de formule (III) :

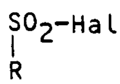

(III)

dans laquelle Hal représente un atome de chlore ou de brome et R conserve la même signification que précédemment, pour obtenir le composé de formule (I) correspondant.

Dans un mode de réalisation préférée du procédé de l'invention, la réaction entre le produit de formule (II) et le produit de formule (III) est effectuée :

a) en présence d'une base forte comme le butyllithium, un hydrure alcalin, comme l'hydrure de sodium ou le bis-(triméthylsilyl) amidure de sodium ;

b) au sein d'un solvant choisi dans le groupe constitué par le tétrahydrofuranne, le benzène, le diméthylformamide, le diméthylsulfoxyde ou l'éther diéthylique du diéthylène glycol.

Les composés de formule (II) utilisés comme produits de départ peuvent être préparés selon le schéma réactionnel suivant :

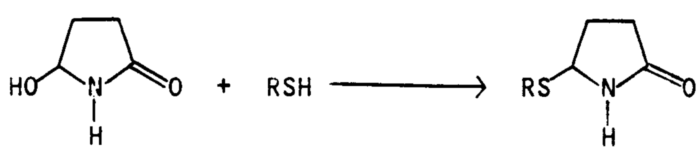

(IV) (II)

Le produit (IV) est décrit dans Heterocycles 22, (8), 1733 (1984).

La partie expérimentale exposée ci-après décrit la préparation de plusieurs produits de formule (II).

Ces produits de formule (II) sont nouveaux et sont en eux-mêmes un des objets de la présente invention.

Les exemples suivants illustrent l'invention sans toutefois la limiter.

**Exemple 1 : 1-benzènesulfonyl 2-oxo 5-éthylthio pyrrolidine.**

A 3,1 g de 5-éthylthio pyrrolidin-2-one dans 130 cm3 de tétrahydrofuranne, on ajoute à -65¤C 14,2 cm3 d'une solution 1,6M de butyllithium dans l'hexane. Après 20 minutes, on ajoute 3,77 g de chlorure de benzènesulfonyle dans 15 cm3 de tétrahydrofuranne. On laisse revenir à température ambiante et concentre à sec sous pression réduite. On obtient 4 g de produit attendu. F = 110-113¤C cristallisé dans l'éthanol.

| Analyse : $C_{12}H_{15}NO_3S_2$ | | | |
|---|---|---|---|
| Calculé :<br>Trouvé : | C% 50,50<br>50,23 | H% 5,30<br>5,35 | N% 4,91<br>4,94 |

**Préparation : 5-éthylthio pyrrolidin-2-one.**

On fait réagir pendant 4 heures à 20¤C un mélange de 2,8 g de 5-hydroxy pyrrolidin-2-one dans 25 cm3 d'éthanethiol avec 1,4 g d'Amberlite® IR 120 H. On dilue le mélange avec de l'éther éthylique, distille le solvant et obtient 2,6 g de produit attendu. F = 65-68¤C que l'on cristallise dans l'éther isopropylique. F = 68-70¤C.

| Analyse : $C_6H_{11}NOS$ | | | |
|---|---|---|---|
| Calculé :<br>Trouvé : | C% 49,62<br>49,85 | H% 7,63<br>7,81 | N% 9,64<br>9,83 |

**Exemple 2 : 1-(4-biphénylyl) sulfonyl 2-oxo 5-éthylthio pyrrolidine.**

A 2,1 g de 5-éthylthio pyrrolidin-2-one en solution dans 155 cm3 de tétrahydrofuranne, on ajoute entre -72¤C et -67¤C 9,6 cm3 de butyllithium en solution 1,5M dans l'hexane. Après 20 minutes à la même température, on ajoute 3,66 g de chlorure de 4-biphénylylsulfonyle dans 20 cm3 de tétrahydrofuranne. On laisse revenir à température ambiante, concentre et chromatographie sur silice (éluant : toluène-acétate d'éthyle 8-2). On obtient 1,7 g de produit attendu. F = 125-128¤C cristallisé de l'isopropanol.

| Analyse : $C_{18}H_{19}NO_3S_2$ | | | |
|---|---|---|---|
| Calculé :<br>Trouvé : | C% 59,81<br>59,77 | H% 5,30<br>5,28 | N% 3,87<br>3,84 |

**Exemple 3 : 1-benzènesulfonyl 2-oxo 5-isopropylthio pyrrolidine.**

A 2,5 g de 5-isopropylthio pyrrolidin-2-one dans 130 cm3 de tétrahydrofuranne, on ajoute à -70¤C, -65¤C 10,48 cm3 d'une solution 1,5M de butyllithium dans l'hexane. On maintient 20 minutes à cette température puis ajoute 2,76 g de chlorure de benzènesulfonyle dans 25 cm3 de tétrahydrofuranne. On laisse revenir à température ambiante et concentre sous pression réduite. On reprend à l'eau, filtre, sèche et obtient 2,15 g de produit attendu. F = 91-93¤C cristallisé de l'éthanol.

| Analyse : $C_{13}H_{17}NO_3S_2$ | | | |
|---|---|---|---|
| Calculé :<br>Trouvé : | C% 52,21<br>51,86 | H% 5,72<br>5,73 | N% 4,68<br>4,62 |

**Préparation : 5-isopropylthio pyrrolidin-2-one.**

On maintient pendant 80 heures à température ambiante un mélange de 9 g de 5-hydroxy pyrrolidin-2-one dans 40 cm3 de 2-propanethiol et 5 g de résine Amberlite® IR 120 H. On dilue à l'éther, filtre, concentre sous pression réduite et obtient 8,5 g de produit attendu. F = 77-79¤C cristallisé de l'éther isopropylique. F = 85-87¤C après recristallisation.

| Analyse : $C_7H_{13}NOS$ | | | |
|---|---|---|---|
| Calculé :<br>Trouvé : | C% 52,80<br>52,56 | H% 8,23<br>8,30 | N% 8,80<br>8,81 |

**Exemple 4 : 1-(3-trifluorométhyl benzène)sulfonyl 2-oxo 5-isopropylthio pyrrolidine.**

A 2,6 g de 5-isopropylthio pyrrolidin-2-one en solution dans 110 cm3 de tétrahydrofuranne, on ajoute à -70¤C 10,2 cm3 d'une solution 1,6M de butyllithium dans l'hexane. Après 20 minutes, on ajoute 4 g de chlorure de 3-trifluorométhyl benzènesulfonyle (J. Org. Chem. 25, 1824, (1960)) dans 20 cm3 de tétrahydrofuranne. On laisse revenir à température ambiante, concentre, lave à l'eau et obtient 3,15 g de produit attendu. F = 105-106¤C cristallisé de l'éthanol.

| Analyse : $C_{14}H_{16}F_3NO_3S_2$ | | | |
|---|---|---|---|
| Calculé :<br>Trouvé : | C% 45,77<br>45,79 | H% 4,39<br>4,36 | N% 3,81<br>3,79 |

**Exemple 5 : 1-(4-nitrobenzène) sulfonyl 2-oxo 5-isopropylthio pyrrolidine.**

A 2,9 g de 5-isopropylthio pyrrolidin-2-one dans 120 cm3 de tétrahydrofuranne, on ajoute à -70¤C 12,1 cm3 d'une solution 1,5M de butyllithium dans l'hexane. Après 20 minutes, on ajoute à -70¤C 4,03 g de chlorure de 4-nitro benzènesulfonyle. On laisse revenir à température ambiante, concentre, lave à l'eau et obtient 3,2 g de produit attendu. F = 125-127¤C cristallisé de l'éthanol.

| Analyse : $C_{13}H_{16}N_2O_5S_2$ | | | |
|---|---|---|---|
| Calculé :<br>Trouvé : | C% 45,33<br>45,26 | H% 4,68<br>4,67 | N% 8,13<br>8,04 |

**Exemple 6 : 1-benzènesulfonyl 2-oxo 4-n-butylthio pyrrolidine.**

A 3,1 g de 5-n-butylthio pyrrolidin-2-one dans 130 cm3 de tétrahydrofuranne, on ajoute à -65¤C/-71¤C 11,2 cm3 d'une solution 1,6M de butyllithium dans l'hexane. Après 20 minutes à la même température, on ajoute 3,16 g de chlorure de benzènesulfonyle dans 20 cm3 de tétrahydrofuranne. On laisse revenir à température ambiante, concentre à sec, chromatogrphie sur silice (éluant : hexane-acétate d'éthyle 1-1) et obtient 3,8 g de produit attendu.

| Analyse : $C_{14}H_{19}NO_3S_2$ | | | |
|---|---|---|---|
| Calculé :<br>Trouvé : | C% 53,65<br>53,91 | H% 6,11<br>6,17 | N% 4,47<br>4,26 |

**Préparation : 5-n-butylthio pyrrolidin-2-one.**

On agite pendant 24 heures un mélange de 3,5 g de 5-hydroxy pyrrolidin-2-one dans 20 cm3 de butanethiol avec 1,75 g de résine Amberlite® IR 120 H. On distille le solvant sous pression réduite, reprend avec de l' hexane, filtre, concentre et chromatographie sur silice (éluant : acétate d'éthyle). On obtient 4,5 g

de produit attendu. Eb : 230-235¤C sous 0,05 mbar.

| Analyse : $C_8H_{15}NOS$ | | | |
|---|---|---|---|
| Calculé :<br>Trouvé : | C% 55,45<br>55,28 | H% 8,72<br>8,78 | N% 8,08<br>8,05 |

**Exemple 7 : 1-benzènesulfonyl 2-oxo 5-propylthio pyrrolidine.**

A 2,3 g de 5-propylthio pyrrolidin-2-one en solution dans 120 cm3 de tétrahydrofuranne, on ajoute à -70¤C/-65¤C 9,6 cm3 d'une solution 1,5M de butyllithium dans l'hexane. Après 20 minutes à cette température, on ajoute 2,55 g de chlorure de benzènesulfonyle dans 20 cm3 de tétrahydrofuranne. On laisse revenir lentement à température ambiante, concentre et chromatographie sur silice (éluant : hexane-acétate d'éthyle 3-7). On obtient 1,5 g de produit attendu. F = 49-51¤C cristallisé de l'éther isopropylique.

| Analyse : $C_{13}H_{17}NO_3S_2$ | | | |
|---|---|---|---|
| Calculé :<br>Trouvé : | C% 52,15<br>52,37 | H% 5,72<br>5,68 | N% 4,68<br>4,77 |

**Préparation : 5-propylthio pyrrolidin-2-one.**

On agite pendant 2 heures 45 minutes à température ambiante, un mélange de 4 g de 5-éthoxy pyrrolidin-2-one, 30 cm3 de propanethiol et 2 g de résine Amberlite®-15. On filtre la résine puis distille le propanethiol en excès. On obtient 2,8 g de produit attendu isolé dans l'éther isopropylique. F = 40-42¤C.

| Analyse : $C_7H_{13}NOS$ | | | |
|---|---|---|---|
| Calculé :<br>Trouvé : | C% 52,80<br>52,86 | H% 8,23<br>8,17 | N% 8,80<br>8,81 |

**Exemple 8 : 1-(4-nitrobenzène) sulfonyl 2-oxo 5-éthylthio pyrrolidine.**

A une solution de 2 g de 5-éthylthio pyrrolidin-2-one dans 110 cm3 de tétrahydrofuranne, on ajoute à -70¤C 9,2 cm3 d'une solution 1,5M de butyllithium dans l'hexane. Après 20 minutes à cette température, on ajoute 3,06 g de chlorure de 4-nitrobenzènesulfonyle en solution dans 10 cm3 de tétrahydrofuranne. On laisse la température remonter lentement, concentre à sec, ajoute de l'eau, filtre, sèche et obtient 3 g de produit attendu. F = 113-115¤C cristallisé dans l'éthanol.

| Analyse : $C_{12}H_{14}N_2O_5S_2$ | | | |
|---|---|---|---|
| Calculé :<br>Trouvé : | C% 43,62<br>43,49 | H% 4,27<br>4,24 | N% 8,48<br>8,39 |

**Exemple 9 : 1-(3-fluorométhyl) benzènesulfonyl 2-oxo 5-éthylthio pyrrolidine.**

A une solution de 2 g de 5-éthylthio pyrrolidin-2-one dans 110 cm3 de tétrahydrofuranne, on ajoute à -70¤C 9,2 cm3 d'une solution 1,5M de butyllithium dans l'hexane. On maintient pendant 20 minutes à cette température puis ajoute 3,36 g de chlorure de 3-trifluorométhyl benzènesulfonyle. On laisse revenir lentement à température ambiante. On concentre à sec, reprend à l'eau, filtre, sèche et obtient 2,8 g de produit attendu. F = 78-79¤C cristallisé de l'éthanol.

| Analyse : $C_{13}H_{14}F_3NO_3S_2$ | | | |
|---|---|---|---|
| Calculé :<br>Trouvé : | C% 44,13<br>44,11 | H% 3,99<br>3,92 | N% 3,96<br>3,87 |

**Exemple 10 : 1-(4-méthoxy) benzènesulfonyl 2-oxo 5-éthylthio pyrrolidine.**

A une solution de 1,85 g de 5-éthylthio pyrrolidin-2-one dans 100 cm3 de tétrahydrofuranne, on ajoute à -70¤C 8,5 cm3 d'une solution 1,5M de butyllithium dans l'hexane. On maintient pendant 15 minutes à cette température puis ajoute 2,65 g de chlorure de 4-méthoxy benzènesulfonyle en solution dans le tétrahydrofuranne. On laisse revenir à température ambiante, concentre à sec, reprend à l'eau, filtre et cristallise 2 fois dans l'isopropanol. On obtient 2,2 g de produit attendu. F = 95-97¤C.

| Analyse : $C_{13}H_{17}NO_4S_2$ | | | |
|---|---|---|---|
| Calculé :<br>Trouvé : | C% 49,50<br>49,63 | H% 5,43<br>5,38 | N% 4,44<br>4,32 |

**Exemples de compositions pharmaceutiques.**

a) On a préparé des comprimés répondant à la formule suivante :

| | |
|---|---|
| - Produit de l'exemple 1<br>- Excipient q.s. pour un comprimé terminé à | 100 mg<br>300 mg |
| (Détail de l'excipient : lactose, amidon de blé, amidon traité, amidon de riz, stéarate de magnésium, talc). | |

b) On a préparé des gélules répondant à la formule suivante :

| | |
|---|---|
| - Produit de l'exemple 2<br>- Excipient q.s. pour une gélule terminée à | 200 mg<br>300 mg |
| (Détail de l'excipient : talc, stéarate de magnésium, Aérosil®). | |

**ETUDE PHARMACOLOGIOUE**

**Toxicité aiguë et comportement des produits de l'invention.**

Nous avons utilisé des souris mâles ($CD_1$ Charles Rivers) d'un poids de 22-23 g à jeun depuis 16 heures. Les produits leur furent administrés normalement par voie orale aux doses de 1000 - 500 - 250 mg/kg.

L'effet des produits sur le comportement des animaux fut évalué suivant la méthode décrite par Irvin (Psychopharmacologia (1968), 13, 222-257) durant les 8 premières heures et à la 24ème heure.

La mortalité fut relevée pendant les 7 jours suivant le traitement.

La $DL_{50}$ a ainsi été trouvée supérieure à 1000 mg/kg pour les produits des exemples 1 et 3.

**Apprentissage et mémorisation.**

Nous avons utilisé des souris mâles ($CD_1$ Charles Rivers) d'un poids de 25-30 g. Les animaux sont placés dans la partie lumineuse d'un box à deux compartiments communicants par une ouverture (G. Galliani, R. Cesana and F. Barzaghi, Med. Sci. Res. 15, 313-314, (1987)).

A l'instant où la souris passe du compartiment lumineux au compartiment obscur, l'ouverture se ferme et elle est immédiatement punie par une décharge électrique aux pattes. L'animal soumis à cette procédure

apprend à mémoriser la punition. En fait, si on le remet dans le compartiment lumineux, il évitera ainsi de franchir l'ouverture et de rentrer dans le compartiment obscur.

Pour induire une amnésie rétrograde, les animaux sont soumis immédiatement après l'apprentissage à un électrochoc. Après l'électrochoc, les produits sont administrés par voie orale aux doses de 12,5 ; 25 ; 50 ; 100 ; 200 et 400 mg/kg.

Nous avons utilisé de 10 à 50 animaux par dose.

L'effet antiamnésique des produits est évalué 3 heures après le traitement, en utilisant le même protocole que celui utilisé pour l'acquisition.

Le temps mis par l'animal pour retourner dans la chambre obscure (temps limite 180 secondes) est utilisé comme paramètre d'évaluation.

Dans les mêmes conditions expérimentales, les animaux témoins entrent avec un laps de temps de 40-50 secondes.

Les produits actifs sont ceux qui provoquent une augmentation significative du temps de latence.

Les résultats sont exprimés en pourcentages d'augmentation du temps de latence par rapport aux témoins correspondants. Des résultats obtenus avec 2 produits de référence sont fournis.

Les résultats sont les suivants :

**Pourcentage d'augmentation du temps de latence par rapport aux témoins**

| Produit de l'exemple | Dose mg/kg os | | | | | |
|---|---|---|---|---|---|---|
| | 400 | 200 | 100 | 50 | 25 | 12,5 |
| 1 | 98* | 151* | 101* | 71* | 50* | 9 |
| 2 | - | 100* | 105* | 45* | 32 | - |
| 3 | 105* | 110* | 79* | 66* | 18 | - |
| 4 | 106* | 77* | 71* | 46 | 21 | - |
| PIRACETAM | - | 20 | 48* | 10 | 19 | - |
| ANIRACETAM | - | 32 | 88* | 77* | 39 | - |

* Valeurs statistiquement différentes par rapport aux témoins.

**Conclusion :**

Les produits des exemples ont montré une activité antiamnésique importante à des doses comprises entre 50 et 200 mg/kg per os et très supérieure à celle des produits de référence.

**Revendications**

**Revendications pour les Etats contractants suivants : CH, DE, FR, GB, IT, LI, NL**

**1.** Les composés répondant à la formule générale (I) :

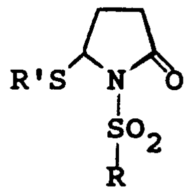

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyrannyle, pyridyle, benzofurannyle, isobenzofurannyle, chromanyle, isochromanyle, chroményle, xanthényle, phénoxathiényle, oxazolyle, isoxazolyle, furazanyle, phénoxazinyle, thiéno[2,3-b]furanyle, 2H-furo[3,2-b]pyrannyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, comme le fluor, le chlore, le brome, les groupements $CF_3$, $SCF_3$, $OCF_3$, $NO_2$, $NH_2$ ou $C{\equiv}N$, le radical phényle, les groupements acyles ou alcoxycarbonyles renfermant de 2 à 8 atomes de carbone et les groupements alcoylsulfonyles renfermant de 1 à 6 atomes de carbone.

**2.** Les composés de formule (I) tels que définis à la revendication 1, dans lesquels R représente un radical phényle éventuellement substitué par l'un des substituants mentionnés dans la revendication 1.

**3.** Les composés de formule (I) tels que définis à la revendication 1, dans lesquels R' représente un radical alcoyle linéaire, ramifié ou cyclique, renfermant jusqu'à 8 atomes de carbone.

**4.** Les composés de formule (I) tels que définis à la revendication 3, dans lesquels R' représente un radical éthyle ou isopropyle.

**5.** Les composés de formule (I) tels que définis à la revendication 1, dont les noms suivent :
- la 1-(benzènesulfonyl) 2-oxo 5-éthylthio pyrrolidine,
- la 1-(benzènesulfonyl) 2-oxo 5-isopropylthio pyrrolidine.

**6.** A titre de médicaments, les composés définis à l'une quelconque des revendications 1 à 4.

**7.** A titre de médicaments, les composés définis à la revendication 5.

**8.** Les compositions pharmaceutiques renfermant comme principe actif au moins un médicament défini à la revendication 6 ou 7.

**9.** Procédé de préparation des composés de formule (I) tels que définis à l'une quelconque des revendications 1 à 5, caractérisé en ce que l'on soumet un composé de formule (II) :

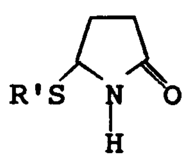

(II)

dans laquelle R' conserve la même signification que précédemment, à l'action d'un composé de formule (III) :

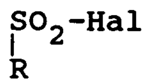

(III)

dans laquelle Hal représente un atome de chlore ou de brome et R conserve la même signification que précédemment pour obtenir le composé de formule (I) correspondant.

**10.** A titre de produits chimiques nouveaux, les composés de formule (II) :

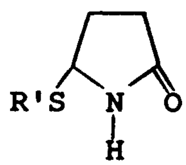

(II)

tels que définis à la revendication 9.

**Revendications pour l'Etat contractant suivant : ES**

**1.** Procédé de préparation des composés répondant à la formule générale (I) :

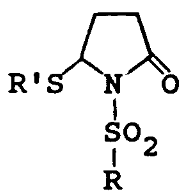

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyrannyle, pyridyle, benzofurannyle, isobenzofurannyle, chromanyle, isochromanyle, chroményle, xanthényle, phénoxathiényle, oxazolyle, isoxazolyle, furazanyle, phénoxazinyle, thiéno[2,3-b]furannyle, 2H-furo[3,2-b]pyrannyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, comme le fluor, le chlore, le brome, les groupements $CF_3$, $SCF_3$, $OCF_3$, $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone, caractérisé en ce que l'on soumet un composé de formule (II) :

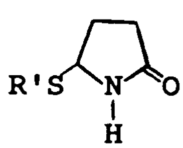

(II)

dans laquelle R' conserve la même signification que précédemment, à l'action d'un composé de formule (III) :

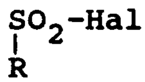

(III)

dans laquelle Hal représente un atome de chlore ou de brome et R conserve la même signification que précédemment pour obtenir le composé de formule (I) correspondant.

**2.** Procédé selon la revendication 1, caractérisé en ce que l'on utilise au départ un composé de formule (III) dans laquelle R représente un radical phényle éventuellement substitué par l'un des substituants indiqués dans la revendication 1.

**3.** Procédé selon la revendication 1 ou 2, caractérisé en ce que l'on utilise au départ un composé de formule (II) dans laquelle R' représente un radical alcoyle linéaire, ramifié ou cyclique, renfermant jusqu'à 8 atomes de carbone.

**4.** Procédé selon la revendication 3, caractérisé en ce que l'on utilise au départ un composé de formule (II) dans laquelle R' représente un radical éthyle ou isopropyle.

**5.** Procédé selon l'une quelconque des revendication 1 à 4, caractérisé en ce que l'on prépare les composés de formule (I), dont les noms suivent :
- la 1-(benzènesulfonyl) 2-oxo 5-éthylthio pyrrolidine,
- la 1-(benzènesulfonyl) 2-oxo 5-isopropylthio pyrrolidine.

**Revendications pour l'Etat contractant suivant : GR**

**1.** Procédé de préparation des composés répondant à la formule générale (I) :

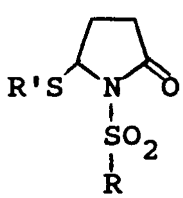

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyrannyle, pyridyle, benzofurannyle, isobenzofurannyle, chromanyle, isochromanyle, chroményle, xanthényle, phénoxathiényle, oxazolyle, isoxazolyle, furazanyle, phénoxazinyle, thiéno[2,3-b]furanyle, 2H-furo[3,2-b]pyranyle, benzoxazolyle ou morpholinyle, le radical R étant éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogène, comme le fluor, le chlore, le brome, les groupements $CF_3$, $SCF_3$, $OCF_3$, $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone, caractérisé en ce que l'on soumet un composé de formule (II) :

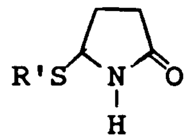

(II)

dans laquelle R' conserve la même signification que précédemment, à l'action d'un composé de formule (III) :

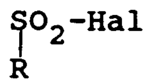

(III)

dans laquelle Hal représente un atome de chlore ou de brome et R conserve la même signification que précédemment pour obtenir le composé de formule (I) correspondant.

**2.** Procédé selon la revendication 1, caractérisé en ce que l'on utilise au départ un composé de formule (III) dans laquelle R représente un radical phényle éventuellement substitué par l'un des substituants indiqués dans la revendication 1.

**3.** Procédé selon la revendication 1 ou 2, caractérisé en ce que l'on utilise au départ un composé de formule (II) dans laquelle R' représente un radical alcoyle linéaire, ramifié ou cyclique, renfermant jusqu'à 8 atomes de carbone.

**4.** Procédé selon la revendication 3, caractérisé en ce que l'on utilise au départ un composé de formule (II) dans laquelle R' représente un radical éthyle ou isopropyle.

**5.** Procédé selon l'une quelconque des revendication 1 à 4, caractérisé en ce que l'on prépare les composés de formule (I), dont les noms suivent :
- la 1-(benzènesulfonyl) 2-oxo 5-éthylthio pyrrolidine,
- la 1-(benzènesulfonyl) 2-oxo 5-isopropylthio pyrrolidine.

**6.** Procédé de préparation de compositions pharmaceutiques caractérisé en ce que l'on met, à titre de principe actif, l'un au moins des produits de formule (I) telle que définie à la revendication 1, sous une forme destinée à cet usage.

**7.** Procédé de préparation de compositions pharmaceutiques caractérisé en ce que l'on met, à titre de principe actif, l'un au moins des produits de formule (I) telle que définie à l'une quelconque des revendications 2 à 4, sous une forme destinée à cet usage.

**8.** Procédé de préparation de compositions pharmaceutiques caractérisé en ce que l'on met, à titre de principe actif, l'un au moins des produits de formule (I) telle que définie à la revendication 5, sous une forme destinée à cet usage.

**Claims**
**Claims for the following Contracting States : CH, DE, FR, GB, IT, LI, NL**

**1.** The compounds corresponding to general formula (I):

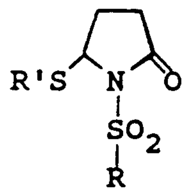

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms, an acyl radical containing 1 to 6 carbon atoms or a benzyl or phenethyl radical and R represents a phenyl or biphenylyl radical or one of the following radicals: furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxasolyl or morpholinyl, the R radical being optionally substituted by one or more substituents chosen from the group constituted by the hydroxy radical, the acetoxy radical, the methoxy radical or the benzyloxy radical, saturated or unsaturated linear, branched or cyclic alkyl radicals containing up to 18 carbon atoms, halogen atoms, such as fluorine, chlorine, bromine, $CF_3$, $SCF_3$, $OCF_3$, $NO_2$, $NH_2$ or C≡N groups, the phenyl radical, acyl or alkoxycarbonyl groups containing 2 to 8 carbon atoms and alkylsulphonyl groups containing 1 to 6 carbon atoms.

**2.** The compounds of formula (I) as defined in claim 1, in which R represents a phenyl radical optionally substituted by one of the substituents mentioned in claim 1.

**3.** The compounds of formula (I) as defined in claim 1, in which R' represents a linear, branched or cyclic alkyl radical containing up to 8 carbon atoms.

**4.** The compounds of formula (I) as defined in claim 3, in which R' represents an ethyl or isopropyl radical.

**5.** The compounds of formula (I) as defined in claim 1, of which the names follow:
- 1-(benzenesulphonyl)-2-oxo-5-ethylthio pyrrolidine,
- 1-(benzenesulphonyl)-2-oxo-5-isopropylthio pyrrolidine.

**6.** As medicaments, the compounds defined in any one of claims 1 to 4.

**7.** As medicaments, the compounds defined in claim 5.

**8.** The pharmaceutical compositions containing as active ingredient at least one medicament defined in claim 6 or 7.

**9.** Preparation process for compounds of formula (I) as defined in any one of claims 1 to 5, characterized in that a compound of formula (II):

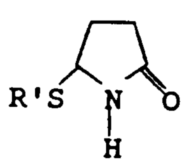

(II)

in which R' keeps the same meaning as previously, is subjected to the action of a compound of formula (III):

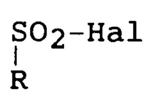

(III)

in which Hal represents a chlorine or bromine atom and R keeps the same meaning as previously, in order to obtain the corresponding compound of formula (I).

**10.** As new chemical products, the compounds of formula (II):

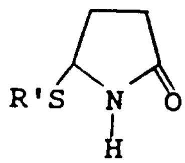

(II)

as defined in claim 9.

**Claims for the following Contracting State : ES**

**1.** Preparation process for compounds corresponding to general formula (I):

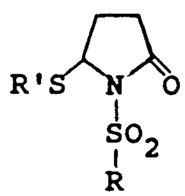

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or biphenylyl radical or one of the following radicals: furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the R radical being optionally substituted by one or more substituents chosen from the group constituted by the hydroxy radical, the acetoxy radical, the methoxy radical or the benzyloxy radical, saturated or unsaturated, linear, branched or cyclic alkyl radical containing up to 18 carbon atoms, halogen atoms, such as fluorine, chlorine, bromine, $CF_3$, $SCF_3$, $OCF_3$, $NO_2$, $NH_2$ or C≡N groups, the phenyl radical, acyl or alkoxycarbonyl groups containing 2 to 8 carbon atoms and alkylsulphonyl groups containing 1 to 6 carbon atoms, characterized in that a compound of formula (II):

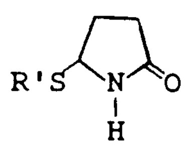

(II)

in which R' keeps the same meaning as previously, is subjected to the action of a compound of formula (III):

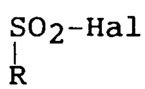

(III)

in which Hal represents a chlorine or bromine atom and R keeps the same meaning as previously, in order to obtain the corresponding compound of formula (I).

**2.** Process according to claim 1, characterized in that at the start a compound of formula (III) is used in which R represents a phenyl radical optionally substituted by one of the substituents indicated in claim

1.

**3.** Process according to claim 1 or 2, characterized in that at the start a compound of formula (II) is used in which R' represents a linear, branched or cyclic alkyl radical containing up to 8 carbon atoms.

**4.** Process according to claim 3, characterized in that at the start a compound of formula (II) is used in which R' represents an ethyl or isopropyl radical.

**5.** Process according to any one of claims 1 to 4, characterized in that the compounds of formula (I) are prepared, the names of which follow:
- 1-(benzenesulphonyl)-2-oxo-5-ethylthio pyrrolidine,
- 1-(benzenesulphonyl)-2-oxo-5-isopropylthio pyrrolidine.

**Claims for the following Contracting State : GR**

**1.** Preparation process for compounds corresponding to general formula (I):

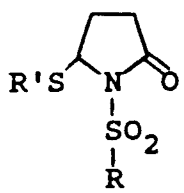

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or biphenylyl radical or one of the following radicals: furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the R radical being optionally substituted by one or more substituents chosen from the group constituted by the hydroxy radical, the acetoxy radical, the methoxy radical or the benzyloxy radical, saturated or unsaturated, linear, branched or cyclic alkyl radical containing up to 18 carbon atoms, halogen atoms, such as fluorine, chlorine, bromine, $CF_3$, $SCF_3$, $OCF_3$, $NO_2$, $NH_2$ or C≡N groups, the phenyl radical, acyl or alkoxycarbonyl groups containing 2 to 8 carbon atoms and alkylsulphonyl groups containing 1 to 6 carbon atoms, characterized in that a compound of formula (II):

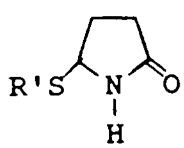

(II)

in which R' keeps the same meaning as previously, is subjected to the action of a compound of formula (III):

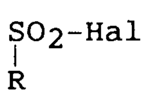

(III)

in which Hal represents a chlorine or bromine atom and R keeps the same meaning as previously, in order to obtain the corresponding compound of formula (I).

**2.** Process according to claim 1, characterized in that at the start a compound of formula (III) is used in

which R represents a phenyl radical optionally substituted by one of the substituents indicated in claim 1.

3. Process according to claim 1 or 2, characterized in that at the start a compound of formula (II) is used in which R' represents a linear, branched or cyclic alkyl radical containing up to 8 carbon atoms.

4. Process according to claim 3, characterized in that at the start a compound of formula (II) is used in which R' represents an ethyl or isopropyl radical.

5. Process according to any one of claims 1 to 4, characterized in that the compounds of formula (I) are prepared, the names of which follow:
   - 1-(benzenesulphonyl)-2-oxo-5-ethylthio pyrrolidine,
   - 1-(benzenesulphonyl)-2-oxo-5-isopropylthio pyrrolidine.

6. Preparation process for pharmaceutical compositions characterized in that at least one of the products of formula (I) as defined in claim 1 is used as active ingredient in a form intended for this use.

7. Preparation process for pharmaceutical compositions characterized in that at least one of the products of formula (I) as defined in any one of claims 2 to 4 is used as active ingredient in a form intended for this use.

8. Preparation process for pharmaceutical compositions characterized in that at least one of the products of formula (I) as defined in claim 5 is used as active ingredient in a form intended for this use.

**Patentansprüche**
**Patentansprüche für folgende Vertragsstaaten : CH, DE, FR, GB, IT, LI, NL**

1. Verbindungen der allgemeinen Formel (I)

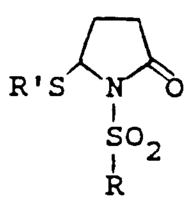

(I)

worin R' für ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl- oder Biphenylylrest oder einen Furyl-, Thienyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest R gegebenenfalls durch einen oder mehrere Substituenten substituiert ist, ausgewählt unter der Hydroxygruppe, dem Acetoxyrest, dem Methoxyrest, dem Benzyloxyrest, den gesättigten oder ungesättigten, linearen, verzweigten oder cyclischen Alkylresten mit bis zu 18 Kohlenstoffatomen, den Halogenatomen, wie Fluor, Chlor, Brom, den Gruppen $CF_3$, $SCF_3$, $OCF_3$, $NO_2$, $NH_2$ oder C≡N, dem Phenylrest, den Acyl- oder Alkoxycarbonylgruppen mit 2 bis 8 Kohlenstoffatomen und den Alkylsulfonylgruppen mit 1 bis 6 Kohlenstoffatomen.

2. Verbindungen der Formel (I), wie in Anspruch 1 definiert, worin R für einen Phenylrest steht, der gegebenenfalls durch einen der in Anspruch 1 genannten Substituenten substituiert ist.

3. Verbindungen der Formel (I), wie in Anspruch 1 definiert, worin R' für einen linearen, verzweigten oder cyclischen Alkylrest mit bis zu 8 Kohlenstoffatomen steht.

4. Verbindungen der Formel (I), wie in Anspruch 3 definiert, worin R' für einen Ethyl- oder Isopropylrest steht.

5. Verbindungen der Formel (I), wie in Anspruch 1 definiert, mit den folgenden Bezeichnungen:
   1-(Benzolsulfonyl)-2-oxo-5-ethylthiopyrrolidin,
   1-(Benzolsulfonyl)-2-oxo-5-isopropylthiopyrrolidin.

6. Als Arzneimittel die Verbindungen gemäß einem der Ansprüche 1 bis 4.

7. Als Arzneimittel die Verbindungen gemäß Anspruch 5.

8. Pharmazeutische Zusammensetzungen, enthaltend als Wirkstoff zumindest ein Arzneimittel, wie in Anspruch 6 oder 7 definiert.

9. Verfahren zur Herstellung der Verbindungen der Formel (I), wie in einem der Ansprüche 1 bis 5 definiert, dadurch gekennzeichnet, daß man eine Verbindung der Formel (II)

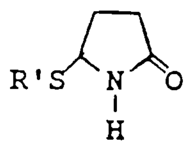

(II)

worin R' die vorstehend angegebene Bedeutung besitzt, der Einwirkung einer Verbindung der Formel (III)

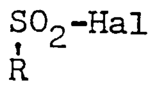

(III)

unterzieht, worin Hal für ein Chlor- oder Bromatom steht und R die vorstehend angegebene Bedeutung besitzt, um zu der entsprechenden Verbindung der Formel (I) zu gelangen.

10. Als neue chemische Produkte die Verbindungen der Formel (II)

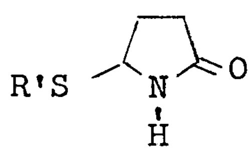

(II)

wie in Anspruch 9 definiert.

**Patentansprüche für folgenden Vertragsstaat: ES**

1. Verfahren zur Herstellung der Verbindungen der allgemeinen Formel (I)

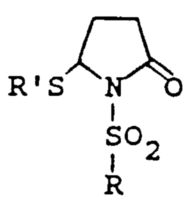

(I)

worin R' für ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl- oder Biphenylylrest oder einen Furyl-, Thienyl-, Pyranyl-, Pyridyl-, Benzofuranyl-, Isobenzofuranyl-, Chromanyl-,

Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest R gegebenenfalls durch einen oder mehrere Substituenten substituiert ist, ausgewählt unter der Hydroxygruppe, dem Acetoxyrest, dem Methoxyrest, dem Benzyloxyrest, den gesättigten oder ungesättigten, linearen, verzweigten oder cyclischen Alkylresten mit bis zu 18 Kohlenstoffatomen, den Halogenatomen, wie Fluor, Chlor, Brom, den Gruppen $CF_3$, $SCF_3$, $OCF_3$, $NO_2$, $NH_2$ oder C≡N, dem Phenylrest, den Acyl- oder Alkoxycarbonylgruppen mit 2 bis 8 Kohlenstoffatomen und den Alkylsulfonylgruppen mit 1 bis 6 Kohlenstoffatomen,
dadurch **gekennzeichnet,** daß man eine Verbindung der Formel (II)

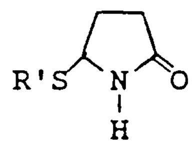

(II)

worin R' die vorstehend angegebene Bedeutung besitzt, der Einwirkung einer Verbindung der Formel (III)

$$\underset{R}{\overset{|}{SO_2}}\text{-Hal}$$

(III)

unterzieht, worin Hal für ein Chlor- oder Bromatom steht und R die vorstehend angegebene Bedeutung besitzt, um zu der entsprechenden Verbindung der Formel (I) zu gelangen.

**2.** Verfahren gemäß Anspruch 1, dadurch gekennzeichnet, daß man von einer Verbindung der Formel (III) ausgeht, worin R für einen Phenylrest steht, der gegebenenfalls durch einen der in Anspruch 1 angegebenen Substituenten substituiert ist.

**3.** Verfahren gemäß Anspruch 1 oder 2, dadurch gekennzeichnet, daß man von einer Verbindung der Formel (II) ausgeht, worin R' für einen linearen, verzweigten oder cyclischen Alkylrest mit bis zu 8 Kohlenstoffatomen steht.

**4.** Verfahren gemäß Anspruch 3, dadurch gekennzeichnet, daß man von einer Verbindung der Formel (II) ausgeht, worin R' für einen Ethyl- oder Isopropylrest steht.

**5.** Verfahren gemäß einem der Ansprüche 1 bis 4, dadurch gekennzeichnet, daß man die Verbindungen der Formel (I) mit den folgenden Bezeichnungen herstellt:
1-(Benzolsulfonyl)-2-oxo-5-ethylthiopyrrolidin,
1-(Benzolsulfonyl)-2-oxo-5-isopropylthiopyrrolidin.

**Patentansprüche für folgenden Vertragsstaat : GR**

**1.** Verfahren zur Herstellung der Verbindungen der allgemeinen Formel (I)

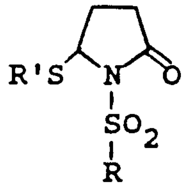

(I)

worin R' für ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6

Kohlenstoffatomen oder einen Benzyl- oder Phenethylrest steht und R einen Phenyl- oder Biphenylylrest oder einen Furyl-, Thienyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest R gegebenenfalls durch einen oder mehrere Substituenten substituiert ist, ausgewählt unter der Hydroxygruppe, dem Acetoxyrest, dem Methoxyrest, dem Benzyloxyrest, den gesättigten oder ungesättigten, linearen, verzweigten oder cyclischen Alkylresten mit bis zu 18 Kohlenstoffatomen, den Halogenatomen, wie Fluor, Chlor, Brom, den Gruppen $CF_3$, $SCF_3$, $OCF_3$, $NO_2$, $NH_2$ oder C≡N, dem Phenylrest, den Acyl- oder Alkoxycarbonylgruppen mit 2 bis 8 Kohlenstoffatomen und den Alkylsulfonylgruppen mit 1 bis 6 Kohlenstoffatomen,
dadurch **gekennzeichnet,** daß man eine Verbindung der Formel (II)

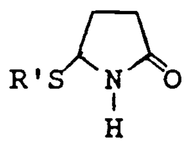

(II)

worin R' die vorstehend angegebene Bedeutung besitzt, der Einwirkung einer Verbindung der Formel (III)

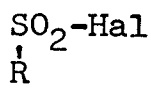

(III)

unterzieht, worin Hal für ein Chlor- oder Bromatom steht und R die vorstehend angegebene Bedeutung besitzt, um zu der entsprechenden Verbindung der Formel (I) zu gelangen.

**2.** Verfahren gemäß Anspruch 1, dadurch gekennzeichnet, daß man von einer Verbindung der Formel (III) ausgeht, worin R für einen Phenylrest steht, der gegebenenfalls durch einen der in Anspruch 1 angegebenen Substituenten substituiert ist.

**3.** Verfahren gemäß Anspruch 1 oder 2, dadurch gekennzeichnet, daß man von einer Verbindung der Formel (II) ausgeht, worin R' für einen linearen, verzweigten oder cyclischen Alkylrest mit bis zu 8 Kohlenstoffatomen steht.

**4.** Verfahren gemäß Anspruch 3, dadurch gekennzeichnet, daß man von einer Verbindung der Formel (II) ausgeht, worin R' für einen Ethyl- oder Isopropylrest steht.

**5.** Verfahren gemäß einem der Ansprüche 1 bis 4, dadurch gekennzeichnet, daß man die Verbindungen der Formel (I) mit den folgenden Bezeichnungen herstellt:
1-(Benzolsulfonyl)-2-oxo-5-ethylthiopyrrolidin,
1-(Benzolsulfonyl)-2-oxo-5-isopropylthiopyrrolidin.

**6.** Verfahren zur Herstellung von pharmazeutischen Zusammensetzungen, dadurch gekennzeichnet, daß man als Wirkstoff zumindest eines der Produkte der Formel (I), wie in Anspruch 1 definiert, in eine für diese Verwendung geeignete Form überführt.

**7.** Verfahren zur Herstellung von pharmazeutischen Zusammensetzungen, dadurch gekennzeichnet, daß man als Wirkstoff zumindest eines der Produkte der Formel (I), wie in einem der Ansprüche 2 bis 4 definiert, in eine für diese Verwendung geeignete Form überführt.

**8.** Verfahren zur Herstellung von pharmazeutischen Zusammensetzungen, dadurch gekennzeichnet, daß man als Wirkstoff zumindest eines der Produkte der Formel (I), wie in Anspruch 5 definiert, in eine für diese Verwendung geeignete Form überführt.